# NEUE EUROPÄISCHE PATENTSCHRIFT

(11) **EP 2 595 942 B2**
(45) Veröffentlichungstag und Bekanntmachung des Hinweises auf die Entscheidung über den Einspruch: **01.03.2017**
(45) Hinweis auf die Patenterteilung: 01.01.2014
(21) Anmeldenummer: 11773396.4
(22) Anmeldetag: 12.10.2011
(51) Int. Cl.: C07C 1/12, B01J 8/08

(54) **VERFAHREN ZUM BEREITSTELLEN EINES METHANREICHEN PRODUKTGASES SOWIE DAZU GEEIGNETE ANORDNUNG**
METHOD FOR PROVIDING A METHANE-RICH PRODUCT GAS, AND AN ARRANGEMENT WHICH IS SUITABLE FOR THIS PURPOSE
PROCÉDÉ DESTINÉ À PRÉPARER UN PRODUIT GAZEUX RICHE EN MÉTHANE ET SYSTÈME APPROPRIÉ

(43) Veröffentlichungstag der Anmeldung: 29.05.2013
(73) Patentinhaber: ETOGAS GmbH, 70565 Stuttgart (DE)
(72) Erfinder: WALDSTEIN, Gregor, 70565 Stuttgart (DE); BUXBAUM, Martin, 6861 Alberschwende (AT)
(74) Vertreter: Leinweber & Zimmermann
(86) Internationale Anmeldenummer: PCT/EP2011/005121
(87) Internationale Veröffentlichungsnummer: WO 2013/053371

(56) Entgegenhaltungen:
- EP-A1- 0 849 245
- EP-B1- 0 326 718
- WO-A2-2010/006386
- WO-A2-2010/006386
- WO-A2-2011/076315
- DE-A1-102009 034 551
- DE-A1-102010 008 857
- US-A- 4 181 675
- US-A- 5 472 986
- 'Modelling of a Chemical Reactor for Simulation of a Methanisation Plant' PROCEEDINGS 8TH MODELICA CONFERENCE 22 März 2011, Seiten 572 - 578
- BAJOHR S. ET AL: 'Speicherung von regenerativ erzeugter elektrischer Energie in der Erdgasinfrastruktur' ERDGAS April 2011, Seiten 200 - 210
- Bio-SNG-Demonstration of the production and utilization of synthetic natural gas(SNG) from solid biofuels Specific Targeted Research or Innovation Project; Seiffert et al. (Dezember 2009)
- SUDIRO M. ET AL: 'Synthetic Natural Gas (SNG) from coal and biomass: a survey of existing process technologies, open issues and perspectives' NATURAL GAS 18 August 2010, Seiten 105 - 126
- KOPYSCINSKI J. ET AL: 'Production of synthetic natural gas (SNG) from coal and dry' FUEL Bd. 89, 06 Februar 2010, Seiten 1763 - 1783
- RÖNSCH S. ET AL: 'Methanisierung von Synthesegasen - Grundlagen und Verfahrensentwicklungen' SYNTHETISCHES ERDGAS August 2011, Seiten 1200 - 1208
- Methanisierung von Ökostrom; Stephan Rieke (Juni 2011)
- Methanisierung - technische Ansätze und deren Bewertung; Bajohr et al. (04.05.2010)
- Einspeisung von Biogas in das Erdgasnetz aus Sicht des (Gasnetzbetreibers) Einspeisers; Leonhard Unterberg (STAWAG) (16.05.2006)

## Beschreibung

Die Erfindung betrifft ein Verfahren zum Bereitstellen eines methanreichen Produktgases, bei dem in einer ersten Betriebsart ein Wasserstoff und Kohlendioxid aufweisendes Eduktgas katalytisch zu einem Gasgemisch methanisiert wird, welches ein vorgegebenes, seine Gaszusammensetzung betreffendes Qualitätskriterium erfüllt und als das Produktgas bereitgestellt wird, und bei dem die katalytische Methanisierung in einer zweiten Betriebsart durch Verringerung, insbesondere Abstellen der Eduktgaszufuhr vermindert, insbesondere gestoppt ist, wobei man einen Übergang zwischen den beiden Betriebsarten in Abhängigkeit einer Verfügbarkeit des Wasserstoffes vornimmt.

Derartige Methanisierungsverfahren sind bekannt und beispielsweise in der WO2011/076315 beschrieben. Die bei der Methanisierung stattfindenden chemischen Reaktionen sind neben den dazu ausgelegten Methanisierungsreaktoren ebenfalls bekannt und werden hier nicht weiter beschrieben. Diesbezüglich wird auf den oben genannten Stand der Technik Bezug genommen. Ebenfalls aus dieser Druckschrift bekannt ist die Nutzung des bereitgestellten Gases in Form einer Einspeisung in ein bestehendes Erdgasnetz. Durch die jeweils herrschenden Einspeisebedingungen wird ein Anforderungsprofil an die Zusammensetzung des einzuspeisenden Produktgases gestellt, welches beispielsweise das Qualitätskriterium bilden kann. In der ersten Betriebsart, beispielsweise dem Normalbetrieb des Methanisierungsreaktors mit einem für seine Auslegung maximalen Gasvolumendurchgang, erfüllt das erzeugte Produktgas das Qualitätskriterium und steht somit zur Einspeisung zur Verfügung.

In der zweiten Betriebsart wird das Verfahren insbesondere dann betrieben, wenn eine für die erste Betriebsart ausreichende Eduktgaszufuhr nicht gegeben oder gewollt ist, konkret wenn Wasserstoff nicht zur Verfügung steht. Dies kann daran liegen, dass zur Erzeugung des Wasserstoffanteils des Eduktgases keine ausreichende elektrische Energie zur Verfügung gestellt wird, beispielsweise bei einer Verringerung von aus erneuerbaren Energiequellen generiertem elektrischen Strom. Insbesondere wird dann in der zweiten Betriebsart der die katalytische Methanisierung bewirkende Reaktor im Standby betrieben, ohne Eduktgaszufuhr. Die Begriffe "Produktgas" und "Eduktgas" sind hier aus Gründen der sprachlichen Einfachheit gewählt, tatsächlich handelt es sich dabei um Gasgemische.

Der Erfindung liegt die Aufgabe zugrunde, ein derartiges intermittierend betriebenes Verfahren weiter zu verbessern.

In verfahrenstechnischer Hinsicht wird diese Aufgabe im wesentlichen durch ein Verfahren gemäß Anspruch 1 gelöst. Es ist vorgesehen, dass ein bei dem Übergang erzeugtes, das Qualitätskriterium nicht erfüllende Gasgemisch im Rahmen des Bereitstellungsverfahrens wenigstens teilweise wiederverwertet wird.

Dabei beruht die Erfindung zunächst auf der Erkenntnis, dass bei diesem Verfahren beim Übergang zwischen den beiden Betriebsarten ein Gasgemisch erzeugt wird, welches das Qualitätskriterium nicht erfüllt. Diese, im weiteren zur sprachlichen Vereinfachung auch als "Schlechtgas" bezeichnete Gasgemisch entsteht beispielsweise beim Übergang von der zweiten Betriebsart (Standby) in die erste Betriebsart, also beim Hochfahren des Methanisierungsreaktors aus dem Bereitschaftszustand heraus, bis der Reaktor hochgefahren ist und stabil ein das Qualitätskriterium erfüllende Gasgemisch erzeugt. Auch beim Übergang von der ersten zur zweiten Betriebsart kann ein Schlechtgas anfallen, wenn der Raum, in welchem die katalytische Methanisierung stattfindet (der Methanisierungsreaktor) mit einem Spülgas, etwa Wasserstoff oder auch dem Produktgas selbst, gespült wird. Wird z.B. mit Wasserstoff gespült (und gleichzeitig die CO₂-Zufuhr gestoppt), ändert sich die stöchiometrische Zusammensetzung des Eduktgases, was rasch die Nichterfüllung des Qualitätskriteriums nach sich zieht. Desweiteren beruht die Erfindung auf der Erkenntnis, dass auch dieses herkömmlich aufgrund seiner Unbrauchbarkeit abgefackelte Schlechtgas noch einen nutzbaren Restwert enthält, und dass sich dieser insbesondere bei einem vergleichsweisen häufigen Umschalten zwischen den beiden Betriebsarten noch technisch sinnvoll nutzen lässt.

Zusätzlich kann auch ein während des zweiten Betriebszustandes, beispielsweise einer Schleichspülung des Reaktionsraumes anfallendes Gas in die Wiederverwertung miteinbezogen werden, wenn nämlich mit Wasserstoff bei geringem Volumenstrom fortlaufend gespült wird. Dabei werden neben dem Austragen von ggf. entstehendem Methan auch ggf. auftretende Gasverluste ausgeglichen.

Der Ausdruck "wenigstens teilweise" umfasst Ausführungen, bei denen ein Teil des Volumens oder das ganze Volumen des Schlechtgases in unmodifizierter Form wiederverwertet wird, aber auch Ausführungen, bei denen ein Schlechtgasanteil in modifizierter Zusammensetzung wiederverwertet wird, sowie Kombinationen dieser Ausführungen.

Erfindungsgemäß erfolgt die Wiederverwertung des Gasgemisches (Schlechtgases) durch dessen (wenigstens teilweise) Rückführung zu einem vor der Methanisierung erfolgenden Verfahrensschritt. Dabei wird ausgenutzt, dass es sich bei dem Schlechtgas um eine Mischung aus Eduktgasbestandteilen und Produktgasbestandteilen handelt.

Erfindungsgemäß wird die Rückführung durch eine Gaszwischenspeicherung zeitlich verzögert. Dies erlaubt eine variablere Gestaltung der Rückführung. Insbesondere kann eine in der ersten Betriebsart vorgenommene Rezirkulation von Produktgas zur Vermeidung von "hot-spots" im Reaktor durch Speisung aus dem Zwischenspeicher ergänzt oder ersetzt werden.

Zweckmäßig wird das rückgeführte Gasgemisch durch Mischung mit einem oder mehreren Eduktgasbestandteilen auf eine zu Beginn der Methanisierung gewünschte Zusammensetzung konditioniert. Dann müssen die Verfahrensparameter der Methanisierung nicht zwangsläufig an die Rückführung angepasst werden, vielmehr wird die Rückführung passend zu den eingestellten Verfahrensparametern modifiziert. Bei der Konditionierung wird auf den Nicht-Methangehalt abgestellt, da sich Methan im Reaktor ähnlich wie ein Inertgas verhält.

In diesem Zusammenhang ist zweckmäßig vorgesehen, dass eine Konditionierung bereits während der Gaszwischenspeicherung erfolgt. Dann/dort lässt sich die Zusammensetzung des gespeicherten Schlechtgases und dessen Modifizierung geeignet messen und steuern.

Eine Konditionierung kann jedoch auch außerhalb des Zwischenspeichers erfolgen. Dabei wird das rückgeführte Gasgemisch durch die Konditionierung im wesentlichen auf die Zusammensetzung des Eduktgases abgestimmt. Somit muss der Methanisierungsvorgang nicht mehr zwischen Eduktgas und konditioniertem Schlechtgas unterscheiden. Ist das gespeicherte Schlechtgas im wesentlichen aus Spülgasbestandteilen wie Wasserstoff gebildet, wird durch Zudosierung von Kohlendioxid im richtigen stöchiometrischen Verhältnis für die Methanisierungsreaktion gesorgt. Alternativ könnte auch die H₂-Zugabe verringert werden.

In einer möglichen Ausführungsform ist die zeitliche Verzögerung der Rückführung aufrechterhalten, bis die Abstimmung der Gasgemischzusammensetzung abgeschlossen ist. Der Zwischenspeicher wird erst dann entleert, wenn sein Inhalt dem Methanisierungsreaktor in der gewünschten Zusammensetzung zuführbar ist.

Besonders bevorzugt wird in der ersten Betriebsart die Eduktgaszufuhr stöchiometrisch und/oder volumenstrommäßig in Abhängigkeit des Volumenstroms des rückgeführten Gasgemisches (Schlechtgases) angepasst. Dazu wird berücksichtigt, dass in der ersten Betriebsart, beispielsweise dem Normalbetrieb bei maximaler Auslastung, die bei der Methanisierung strömende Gasmenge im wesentlichen konstant bleiben soll, so dass für die Phase der Rückführung die Eduktgaszufuhr entsprechend verringert wird. Dies kann unabhängig von einer etwaigen zusätzlichen Rückführung von Produktgas erfolgen, welche aus Gründen der Verfahrensführung in der ersten Betriebsart zusätzlich durchgeführt wird. Letztere dient allerdings z.B. der Verringerung der Reaktivität des die Methanisierungsstrecke, insbesondere an derem Beginn am Reaktoreingang durchlaufenden Gasgemisches, wozu ein Teil des hergestellten Produktgases auch dann rückgeführt wird, wenn es bereits das Qualitätskriterium erfüllt. Erforderliche Parameter des rückgeführten Gasgemisches (Schlechtgases/konditionierten Schlechtgases), insbesondere Druck und/oder Temperatur werden passend für die Zudosierung eingestellt.

Grundsätzlich unterliegt das erfindungsgemäße Verfahren keinen Einschränkungen dahingehend, aus welcher Quelle das Kohlendioxid des Eduktgases bezogen wird. Beispielsweise wird dieser Eduktgasbestandteil über eine Biogasaufbereitung, insbesondere mittels Aminwäsche von einer Biogasanlage bereitgestellt. So kann auch eine anderweitig anfallende Freisetzung von CO₂ in die Atmosphäre verhindert werden. Denn das CO₂ wird in diesem Verfahren als wertvolle Rohstoffquelle angesehen.

Ebenfalls ist vorgesehen, dass zur Wiederverwertung ein nach der Abtrennung in der Biogasaufbereitungsanlage erfolgtes und bereitgestelltes Biomethan beiträgt. Auf diese Weise kann ein Methangehalt des Schlechtgases in einfacher Weise wiederverwertet werden und auch während der zweiten Betriebsart bereitgestellt werden.

In einer weiteren bevorzugten Verfahrensgestaltung erfolgt die Wiederverwertung teilweise durch Abtrennen eines das Qualitätskriterium erfüllenden Anteils und dessen Bereitstellung eines Produktgases. Diese Variante eignet sich besonders in jenen Fällen, in denen die Gaszusammensetzung bereits in der Nähe der Erfüllung des Qualitätskriteriums liegt und eine vergleichsweise große Menge Produktgas durch eine relativ geringe Menge Eduktgasreste zu Schlechtgas entwertet ist. Der dabei abgetrennte Teil des Schlechtgases kann entweder über Fackel entsorgt oder im Rahmen einer der übrigen Ausführungsformen wiederverwertet werden.

Gemäß einer nochmals weiteren Ausgestaltung läßt sich das Schlechtgas in eine Gasverwertungseinheit einspeisen, beispielsweise einen Gaskessel oder ein Blockheizkraftwerk.

Die bislang beschriebenen Verfahrensvarianten können ohnehin alternativ aber auch gemeinschaftlich zum Einsatz kommen. Dabei kann vorgesehen werden, dass die Wiederverwertung des Schlechtgases nicht nur teilweise, sondern vollständig erfolgt. Andererseits kann auch vorgesehen werden, die Wiederverwertung des Schlechtgases insoweit vollständig erfolgen zu lassen und eine Freisetzung oder ein Abfackeln des Schlechtgases nur im Ausmaße durchzuführen, in welchem anderweitig die Aufnahmekapazität des Verfahrens für die Wiederverwertung/Rückführung überschritten würde.

Das Qualitätskriterium wird in der Regel vom Verwendungszweck des hergestellten Produktgases abhängen. Erfindungsgemäß bevorzugt ist vorgesehen, dass das Qualitätskriterium ein oder mehrere Subkriterien aufweisen kann. Dazu zählen neben einem Mindestmethangehalt von bevorzugt wenigstens 90%, weiter bevorzugt wenigstens 92%, insbesondere wenigstens 95% als weitere Subkriterien noch ein Höchstkohlendioxidgehalt und/oder ein Höchstwasserstoffgehalt in Frage. Von diesen sollte ersterer höchstens 12%, weiter bevorzugt höchstens 8%, insbesondere höchstens 4% betragen, während letzterer bevorzugt höchstens 4%, weiter bevorzugt höchstens 2%, insbesondere höchstens 1 % betragen soll.

Grundsätzlich ist das erfindungsgemäße Verfahren unabhängig davon, aus welcher Quelle der Wasserstoff für das Eduktgas gewonnen wird. Bevorzugt wird jedoch die elektrolytische Wasserstofferzeugung mittels elektrischer Energie. Vom Betrachtungspunkt der Stromnetzdienstleistung bringt die verwendete Elektrolyseeinheit eine erforderliche Netzdienstleistung in Form eines Verbrauchers (Last) und stellt Wasserstoff bereit. In diesem Zusammenhang wird die elektrische Energie zur Wasserstofferzeugung besonders bevorzugt aus regenerativen Energiequellen, insbesondere den zeitlich variabel verfügbaren Energiequellen wie Wind- und/oder Sonnenenergie stammen, welche insbesondere zur erfindungsgemäßen Konvertierung in das Produktgas als chemischen Energieträger geeignet nutzbar sind. Geeignet sind ansonsten auch andere Verfahren, bei denen Wasserstoff intermittierend abfällt.

Je nach Verfügbarkeit solcher Energiequellen wird das Umschalten zwischen den Betriebsarten durchgeführt, wobei die zweite Betriebsart angesteuert wird, wenn nur noch wenig oder keine elektrische Energie mehr zur Verfügung steht. Der sich auf diese Weise ergebende intermittierende Betrieb wird dann insbesondere derart durchgeführt, dass ein Übergang zwischen den Betriebsarten durchschnittlich wenigstens im Bereich von mehrmals pro Woche, insbesondere wenigstens einmal pro Tag erfolgt.

In vorrichtungstechnischer Hinsicht wird die Aufgabe gelöst durch eine Anordnung gemäß Anspruch 15. Vorgesehen ist somit eine Anordnung zum Bereitstellen eines methanreichen Produktgases mit einem katalytisch wirkenden Methanisierungsreaktor, einer dem Methanisierungsreaktor vorgeschalteten Zufuhreinrichtung zum Einspeisen eines Wasserstoff und Kohlendioxid aufweisenden Eduktgases in den Reaktor, und einen dem Reaktor nachgeschalteten Gasausgang zur Bereitstellung des in dem Reaktor erzeugten und ein vorgegebenes, die Gaszusammensetzung betreffendes Qualitätskriterium erfüllenden Gasgemisches als das Produktgas, weiter aufweisend eine zwischen dem Reaktorausgang und dem Gasausgang ankoppelnde Verwertungseinrichtung zur Auskopplung und wenigstens teilweise Wiederverwertung eines aus dem Reaktor austretenden und das Qualitätskriterium nicht erfüllenden Gasgemisches.

Die Vorteile der erfindungsgemäßen Anordnung ergeben sich im wesentlichen aus der obigen Beschreibung des erfindungsgemäßen Verfahrens. So weist die Verwertungseinrichtung eine Rückführeinrichtung zur wenigstens teilweisen Rückführung des das Qualitätskriterium nicht erfüllenden Gasgemisches (Schlechtgases) an eine vor dem Reaktoreingang gelegene Zufuhrstelle auf. Ebenfalls ist vorgesehen, einen Zwischenspeicher für die Rückführeinrichtung vorzusehen, und ggf. eine Leitungsanordnung zur den Reaktor umgehenden Zufuhr wenigstens eines Eduktgasbestandteils in den Zwischenspeicher.

Die Leitungsanordnung kann dabei eine getrennte Zuleitung aus der H₂ und/oder eine getrennte Zuleitung aus der CO₂-Quelle der Zufuhreinrichtung aufweisen.

Grundsätzlich ist es denkbar, dass der Reaktor auf verschiedene Verfahrensbedingungen getestet und die Produktgaszusammensetzung bei feststehenden Verfahrensparametern aus den Testreihen bekannt ist. Es kann jedoch auch vorgesehen werden, die Gaszusammensetzungen durch entsprechende Messeinrichtungen festzustellen, zum einen am Reaktorausgang oder jedenfalls vor dem Gasausgang, aber auch im Zwischenspeicher selbst.

Die Rückführeinrichtung kann auch einen Ausgleichsraum zum Ausgleich zeitlich variabler Gasvolumenströme aufweisen. Dies ist besonders in einer Ausführungsform von Vorteil, in welcher das ausgekoppelte Schlechtgas fortlaufend unmittelbar zum Reaktoreingang rezirkuliert wird, solange bis das aus dem Reaktor austretende Gas das Qualitätskriterium erfüllt. Dabei wird der entstehende Gasvolumenstrom schwanken, und die Schwankungen können durch den Ausgleichsraum abgemildert oder ausgeglichen werden.

Desweiteren kann je nach bevorzugter Verfahrensgestaltung die Verwertungseinrichtung auch eine Gastrenneinrichtung zum Abtrennen eines das Gasgemisch hinsichtlich des Qualitätskriteriums verschlechternden Gasanteils aufweisen. Hierzu bieten sich Gaswäschen wie die Aminwäsche oder Membranverfahren an, mit denen beispielsweise der Kohlendioxidgehalt des Schlechtgases verringert werden kann. Auch kann eine Zuleitung zu einer Gasverwertungseinrichtung Bestandteil der Anordnung sein.

Zur Steuerung der Anordnung ist eine Steuereinrichtung vorgesehen, die zur Steuerung gemäß einem der oben beschriebenen Verfahrensaspekte ausgelegt ist.

Weitere Merkmale, Einzelheiten und Vorteile der Erfindung ergeben sich aus der folgenden Beschreibung beispielhafter Ausführungsformen, von denen
- Fig. 1: schematisch eine erste Ausführungsform der Erfindung zeigt,
- Fig. 2: schematisch eine zweite Ausführungsform der Erfindung zeigt.

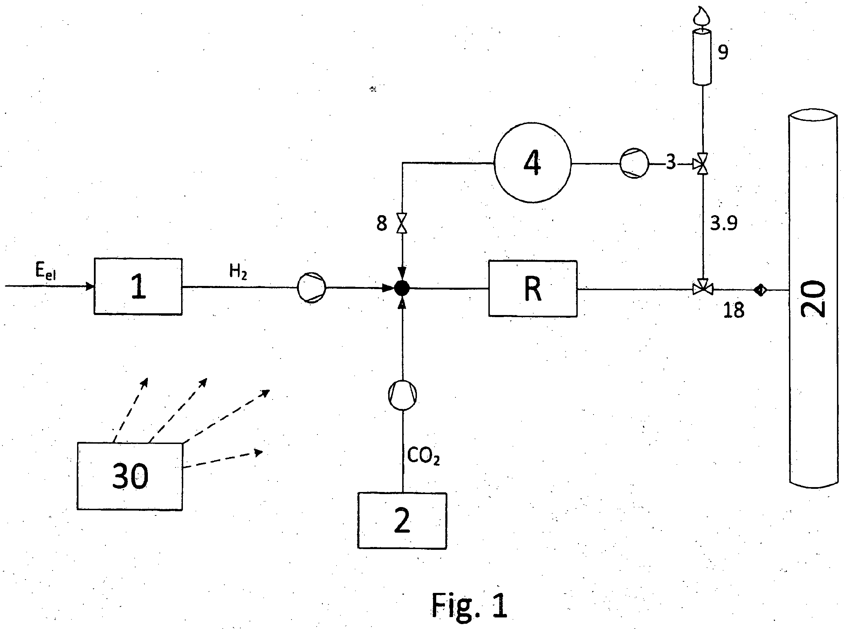

Zentraler Bestandteil der in Fig. 1 schematisch gezeichneten Darstellung eines Methanisierungsverfahrens ist der Reaktor R, in welchem die katalytische Methanisierung des zugeführten Eduktgases abläuft. Der Reaktor R kann ein- oder mehrstufig, z.B. zweistufig ausgeführt werden. Der Reaktoreingang R wird mit einem Eduktgas gespeist, welches Wasserstoff H₂ und Kohlendioxid CO₂ in zur Methanisierung richtigen stöchiometrischen Idealverhältnis aufweist, wobei von diesem Idealverhältnis auch in gewissem Maße abgewichen werden kann, um das Reaktionsgleichgewicht im Reaktor R je nach gewünschtem hergestellten Produktgas zu verschieben. So kann von dem Verhältnis H zu CO₂ von 4:1 abgewichen werden, um eine Zusammensetzung von z.B. 92% CH₄, 4% CO₂, 4% Wasserstoff am Reaktorausgang zu erhalten. Dann hat der Eduktgasrest nicht die Stöchiometrie des Eduktgases. Mit der anfänglichen Abweichung läßt sich ein erhöhter Methangehalt erreichen. Man paßt sich den Anforderungen des Gasnetzes optimal an.

Der Kohlendioxidanteil wird von einer Kohlendioxidquelle 2 bereitgestellt, welche z.B. eine Aminwäsche 12 einer Biogasanlage sein kann.

Der Wasserstoff H₂ wird in diesem Ausführungsbeispiel elektrolytisch von einer Elektrolyseeinheit 1 erzeugt. Die dazu erforderliche elektrische Energie E_{EL} wird in dieser Ausführungsform aus dem Stromnetz bezogen. Dabei wird bevorzugt Strom bezogen, welcher bezüglich des Stromnetzdienstes einer Stromeinspeisung zugeordnet ist, welche aus erneuerbaren Energiequellen stammt und aktuell im Stromnetz nicht abgesetzt werden kann, beispielsweise Windenergie. Ein alternatives Beispiel für eine intermittierend wirkende H₂-Quelle wäre etwa eine konstante H₂ Produktion, z.B. bei der Cl-Gewinnung nebst wechselndem Bezug von H₂ durch ein Kraftwerk und somit H₂-Verfügbarkeit, wenn das Kraftwerk nicht bezieht.

Im Normalbetrieb bei zugeführter elektrischer Energie und deren Wandlung in Wasserstoff durch die Elektrolyseeinheit 1 wird der Reaktor R in einer Auslastung betrieben, für die er maximal ausgelegt ist, und das im Reaktor R erzeugte Gasgemisch wird dem Gasausgang 18 zugeleitet. Es erfüllt die für die Einspeisung in das Gasnetz 20 erforderlichen Anforderungen an die Zusammensetzung und weist in diesem Ausführungsbeispiel neben unvermeidbaren Restbestandteilen in Geringstkonzentrationen ca. 92% CH₄, 4% H₂ und 4% CO₂ auf. Soweit erforderlich, wird das Produktgas vor der Einspeisung noch hinsichtlich Brennwert konditioniert und odoriert. Dies ist dem Fachmann allerdings wohl bekannt und nicht zentraler Bestandteil der Erfindung.

Vor dem Gasausgang 18 ist ein Abschnitt 3.9 einer Leitung 3 angekoppelt, über welche das im Reaktor erzeugte Gasgemisch ausgekoppelt und nicht dem Gasausgang 18 zugeführt wird, sofern es den gewünschten Anforderungen (dem Qualitätskriterium) nicht genügt. Herkömmlich würde dieses Schlechtgas dann über eine Fackel 9 entsorgt.

Ein solches Schlechtgas entsteht beispielsweise bei dem intermittierend betriebenen Reaktor R dann, wenn nach einer Phase, in der keine elektrische Energie E_{EL} für die Elektrolyseeinheit 1 bereitgestellt wurde und der Reaktor R in Standby geschaltet wurde, der Reaktor R wieder mit Eduktgas beschickt wird, welcher in der Anlaufphase zum stationären Normalbetrieb, also beim Übergang zwischen dem Standby und dem Normalbetrieb, noch keine ausreichende Methanisierung erreicht.

In dieser Ausführungsform wird dieses Schlechtgas nicht über die hier für Notfälle dennoch vorgesehene Fackel 9 abgebrannt, sondern weiter über die Leitung 3 in einen Zwischenspeicher 4 geleitet und durch Sperrung des nachgeschalteten Ventils 8 darin zwischengespeichert, bis der Reaktor R stationär läuft und spezifikationsgerecht produziert. Dann wird das Ventil 8 geöffnet und der Speicherinhalt des Zwischenspeichers 4 langsam entleert und dem zugeführten Eduktgasstrom beigemengt, so dass es über diese Rückführung einer weitergehenden Methanisierung unterzogen wird und schließlich vollständig wiederverwertet wird. Die Zudosierung kann in einem solch geringen Maß erfolgen, dass sich die dadurch modifizierte Eduktgaszusammensetzung gegenüber der Eduktgaszusammensetzung ohne Rückführung des Schlechtgases für den Reaktor R nur unmerklich unterscheidet und daher die Betriebseinstellungen des Reaktors R nicht geändert werden müssen. Gegebenenfalls werden dazu die Eduktgasquellen einzeln angesteuert, um gezielt mehr oder weniger H₂ bzw. CO₂ einzuspeisen.

Auch beim Übergang von der ersten Betriebsart (dem Normalbetriebszustand) in den Standby kann ein Schlechtgas auftreten und rückgeführt werden. So wird der Reaktor R beispielsweise nach dem Herunterfahren und dem Anhalten der Kohlendioxidzufuhr mit Wasserstoff H₂ gespült. Das Spülgas sowie die vom Spülgas verdrängte Restgasmenge aus dem Reaktor bildet ein Schlechtgas mit vergleichsweise höherem Wasserstoffgehalt. Auf diese Weise kann der Zwischenspeicher 4 auch mit einem Schlechtgas befüllt sein, dessen Verhältnis von Wasserstoff zu Kohlendioxid größer ist als das für die Methanisierungsreaktion ideale stöchiometrische Verhältnis. In diesem Fall ist bei der Zusammenführung des Schlechtgases aus dem Zwischenspeicher 4 mit dem Wasserstoff der Elektrolyseeinheit 1 und dem Kohlendioxid der Kohlendioxidquelle 2 der Wasserstoffzustrom entsprechend reduzierend anzupassen. Eine Befüllung des Zwischenspeichers 4 kann beispielsweise folgendermaßen erfolgen. Beim Abfahren wird mit reinem H₂ gespült. Der damit gefüllte Speicher bleibt stehen, bis der Reaktor das nächste Mal hochfährt. Dann kommt das Schlechtgas aus dem Hochfahrprozeß dazu. Sobald der Reaktor eine gewünschte Einspeisequalität erzeugt, wird mit dem Entleeren des Speichers begonnen.

Die Entleerung des Speichers soll möglichst rasch erfolgen, damit dieser für einen erneuten Übergang wieder zur Verfügung steht. Dazu kann die Eduktgaszufuhr bzw. die der einzelnen Komponenten auch gestoppt bzw. vermindert werden.

Desweiteren ist in Fig. 1 noch schematisch eine Steuereinrichtung 30 eingezeichnet, welche das Gesamtverfahren steuert und dazu entsprechende, nicht dargestellte Steuerleitungen aufweist.

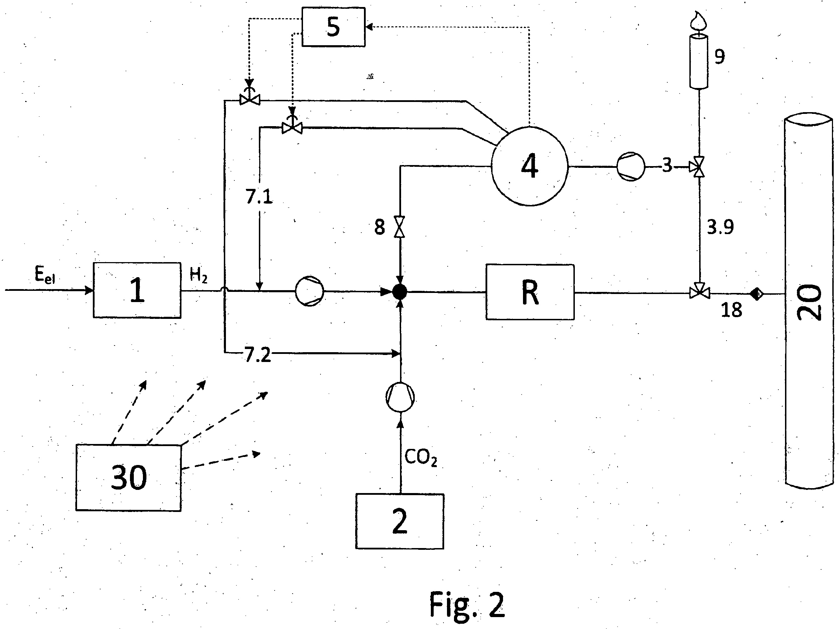

Eine in Fig. 2 dargestellte zweite Ausführungsform stellte eine Modifizierung der ersten Ausführungsform aus Fig. 1 dahingehend dar, dass eine Modifizierung des ausgekoppelten und über die Leitung 3 in den Zwischenspeicher 4 geleiteten Schlechtgases bereits im Zwischenspeicher 4 erfolgen kann. Dazu ist ein Leitungssystem mit einer H₂-Leitung 7.1 und einer CO₂-Leitung 7.2 vorgesehen, über welche in Abhängigkeit der Gaszuammensetzung im Zwischenspeicher 4 von einer diese messenden Messeinrichtung 5 gesteuert Wasserstoff H₂ aus der Elektrolyseeinheit 1 und/oder Kohlendioxid aus der Kohlendioxidquelle 2 gezielt und separat dem Zwischenspeicher 4 zur Mischung mit dem darin enthaltenen Schlechtgas zuführbar ist/sind. Beispielsweise kann auf diese Weise eine Gaszusammensetzung im Zwischenspeicher 4 erzeugt werden, welche sich im wesentlichen wie ein Gemisch aus Eduktgas in der gewünschten Stöchiometrie und einspeisefähigem Produktgas verhält. Wenn der Zwischenspeicherinhalt der Eduktgasspezifikation entspricht, kann der Zwischenspeicher zum Eingang des Reaktors R hin entleert werden.

Diese Konditionierung kann auch in Form einer Vorstufe erfolgen, während dennoch über die Eduktgas-Hauptzuführung je nach Regelung der Anlage nachkonditioniert wird.

Die in den Figuren gezeigten Ausführungsbeispiele sind daher nicht als Einschränkung der Erfindung auszulegen. Vielmehr können die einzelnen in der Beschreibung und den Ansprüchen offenbarten Merkmale sowohl einzeln als auch in ihrer Kombination für die Verwirklichung der Erfindung in ihren verschiedenen Ausführungsformen wesentlich sein.

## Patentansprüche

1. Verfahren zum Bereitstellen eines methanreichen Produktgases, bei dem in einer ersten Betriebsart ein Wasserstoff und Kohlendioxid aufweisendes Eduktgas katalytisch zu einem Gasgemisch methanisiert wird, welches ein vorgegebenes, seine Gaszusammensetzung betreffendes Qualitätskriterium erfüllt und als das Produktgas bereitgestellt wird, und bei dem die katalytische Methanisierung in einer zweiten Betriebsart durch Verringerung, insbesondere Abstellen der Eduktgaszufuhr vermindert, insbesondere gestoppt ist, wobei man einen Übergang zwischen den beiden Betriebsarten in Abhängigkeit einer Verfügbarkeit des Wasserstoffes vornimmt,
**dadurch gekennzeichnet, dass**
ein bei dem Übergang erzeugtes, das Qualitätskriterium nicht erfüllende Gasgemisch im Rahmen des Bereitstellungsverfahrens durch seine wenigstens teilweise Rückführung zu einem vor der Methanisierung erfolgenden Verfahrensschritt wenigstens teilweise wiederverwertet wird, wobei die Rückführung durch eine Gaszwischenspeicherung zeitlich verzögert wird.

2. Verfahren nach Anspruch 1, bei dem das rückgeführte Gasgemisch durch Mischung mit einem oder mehreren Eduktgasbestandteilen auf eine zu Beginn der Methanisierung gewünschte Zusammensetzung konditioniert wird.

3. Verfahren nach Anspruch 2, bei dem die Konditionierung während der Gaszwischenspeicherung erfolgt.

4. Verfahren nach Anspruch 3, bei dem der Nicht-Methananteil des rückgeführten Gasgemisches durch die Konditionierung im Wesentlichen auf die Zusammensetzung des Eduktgases abgestimmt wird.

5. Verfahren nach Anspruch 4, bei dem die zeitliche Verzögerung aufrechterhalten wird, bis die Abstimmung der Gasgemischzusammensetzung abgeschlossen ist.

6. Verfahren nach einem der Ansprüche 1 bis 5, bei dem in der ersten Betriebsart die Eduktgaszufuhr stöchiometrisch und/oder volumenstrommäßig in Abhängigkeit der Zusammensetzung und/oder des Volumenstroms des rückgeführten Gasgemisches angepasst wird.

7. Verfahren nach einem der vorhergehenden Ansprüche, bei dem wenigstens ein Eduktgasbestandteil von einer Biogasanlage bereitgestellt wird.

8. Verfahren nach Anspruch 7, bei dem der aus der Biogasanlage bereitgestellte Eduktgasbestandteil aus einem bei einer Biogasaufbereitung, insbesondere mittels Aminwäsche, abgetrennten sehr kohlenstoffreichen Gas gebildet ist.

9. Verfahren nach einem der vorhergehenden Ansprüche, bei dem die Wiederverwertung teilweise durch Abtrennen eines das Qualitätskriterium erfüllenden Anteils und dessen Bereitstellung als Produktgas und/oder durch Speisung des das Qualitätserfordernis nicht erfüllenden Gasgemisches in eine Gasverwertungseinheit, insbesondere einen Gaskessel oder ein Blockheizkraftwerk erfolgt.

10. Verfahren nach einem der vorhergehenden Ansprüche, bei dem die Wiederverwertung des das Qualitätskriterium nicht erfüllenden Gasgemisches mit Ausnahme einer Auskopplung andernfalls die Aufnahmekapazität des Verfahrens überschreitenden Gasmenge vollständig erfolgt, insbesondere gänzlich vollständig erfolgt.

11. Verfahren nach einem der vorhergehenden Ansprüche, bei dem das Qualitätskriterium ein oder mehrere Subkriterien aufweist, die aus der Gruppe:
i) Mindestmethangehalt, insbesondere bevorzugt wenigstens 90%, weiter bevorzugt wenigstens 92%, insbesondere wenigstens 95%,
ii) Höchstkohlendioxidgehalt, insbesondere bevorzugt höchstens 12%, weiter bevorzugt höchstens 8%, insbesondere höchstens 4%,
iii) Höchstwasserstoffgehalt, insbesondere bevorzugt höchstens 4%, weiter bevorzugt höchstens 2%, insbesondere höchstens 1%
gewählt sind.

12. Verfahren nach einem der vorhergehenden Ansprüche, bei dem wenigstens ein Teil des Wasserstoffes des Eduktgases durch Elektrolyse erzeugt wird.

13. Verfahren nach einem der vorhergehenden Ansprüche, bei dem die elektrische Energie zur Wasserstofferzeugung aus im Stromnetz auftretenden Überschussströmen und/oder regenerativen Energiequellen, insbesondere zeitlich variabel verfügbaren Energiequellen wie Wind- und/oder Sonnenenergie stammt.

14. Verfahren nach einem der vorhergehenden Ansprüche, bei dem der Übergang zwischen den Betriebsarten durchschnittlich wenigstens im Bereich von mehrmals pro Woche, insbesondere einmal oder auch mehrmals pro Tag erfolgt.

15. Anordnung zum Bereitstellen eines methanreichen Produktgases mit:
einem katalytisch wirkenden Methanisierungsreaktor (R),
einer dem Methanisierungsreaktor vorgeschalteten Zufuhreinrichtung (1, 2) zum Einspeisen eines Wasserstoff und Kohlendioxid aufweisenden Eduktgases in den Reaktor (R), und
einen dem Reaktor (R) nachgeschalteten Gasausgang (18) zur Bareitstellung des in dem Reaktor (R) erzeugten und ein vorgegebenes, die Gaszusammensetzung betreffendes Qualitätskriterium erfüllenden Gasgemisches als das Produktgas,
**gekennzeichnet durch** eine zwischen dem Reaktorausgang und dem Gasausgang ankoppelnde Verwertungseinrichtung (3, 4; 3, 6; 13) zur Auskopplung und wenigstens teilweise Wiederverwertung eines aus dem Reaktor (R) austretenden und das Qualitätskriterium nicht erfüllenden Gasgemisches, wobei die Verwertungseinrichtung eine Rückführeinrichtung (3, 4; 3, 6) zur wenigstens teilweisen Rückführung des das Qualitätskriterium nicht erfüllenden Gasgemisches an eine vor dem Reaktoreingang gelegene Zufuhrstelle aufweist und wobei die Rückführeinrichtung einen Zwischenspeicher (4) aufweist sowie **durch** eine Steuereinrichtung (30), die die Anordnung zur Ausführung eines Verfahrens nach einem der Ansprüche 1 bis 14 steuert.

16. Anordnung nach Anspruch 15, bei der die Rückführeinrichtung eine Leitungsanordnung (7) zur den Reaktor umgehenden Zufuhr wenigstens eines Eduktgasbestandteils in den Zwischenspeicher aufweist.

17. Anordnung nach Anspruch 16, bei der die Leitungsanordnung eine getrennte Zuleitung (7.1) aus der H₂ und/oder eine getrennte Zuleitung (7.2) aus der CO₂-Quelle der Zufuhreinrichtung aufweist.

18. Anordnung nach einem der Ansprüche. 15 bis 17, bei der die Rückführeinrichtung eine die Gaszusammensetzung im Zwischenspeicher messende Messeinrichtung (5) aufweist.

19. Anordnung nach einem der Ansprüche 15 bis 18, bei dem die Verwertungseinrichtung eine Gastrenneinrichtung (13) zum Abtrennen eines das Gasgemisch hinsichtlich des Qualitätskriteriums verschlechternden Gasanteils aufweist.

20. Anordnung nach einem der Ansprüche 15 bis 19, bei der ein Anschluß für eine Zuleitung (101) zu einer Gasverwertungseinheit (100) vorgesehen ist, insbesondere auch die Zuleitung (101) und/oder die Gasverwertungseinheit (100) selbst.

## Claims

1. A method for providing a methane-rich product gas, wherein in a first operating mode a reactant gas having hydrogen and carbon dioxide is catalytically methanated into a gas mixture which satisfies a specified quality criterion relating to its gas composition and is provided as the product gas, and wherein the catalytic methanation is reduced, in particular stopped, in a second operating mode by reducing, in particular cutting off the reactant gas feed, a transition being carried out between the two operating modes dependently upon an availability of the hydrogen,
**characterised in that**
a gas mixture produced upon the transition, and which does not satisfy the quality criterion, is at least partly recycled within the framework of the providing method by its at least partial refeed to a procedural step taking place before the methanation, wherein the refeed is temporally delayed by intermediate gas storage.

2. The method according to claim 1, wherein the refed gas mixture is conditioned by mixing with one or a number of reactant gas components to form a desired composition at the start of the methanation.

3. The method according to claim 2, wherein the conditioning takes place during the intermediate gas storage.

4. The method according to claim 3, wherein the non-methane portion of the refed gas mixture is matched substantially to the composition of the reactant gas by the conditioning.

5. The method according to claim 4, wherein the time delay is maintained until the matching of the gas mixture composition is complete.

6. The method according to any of claims 1 to 5, wherein in the first operating mode the reactant gas feed is matched stoichiometrically and/or according to the volumetric flow depending on the composition and/or the volumetric flow of the refed gas mixture.

7. The method according to any of the preceding claims, wherein at least one reactant gas component is provided by a biogas plant.

8. The method according to claim 7, wherein the reactant gas component provided by the biogas plant is formed from a very carbon-rich gas separated during a biogas preparation, in particular by means of amine scrubbing.

9. The method according to any of the preceding claims, wherein the recycling is partially or totally implemented by separating a portion satisfying the quality criterion and by providing it as a product gas and/or by feeding the gas mixture not satisfying the quality requirement into a gas utilisation unit, in particular a gas boiler or a block heat and power plant.

10. The method according to any of the preceding claims, wherein the recycling of the gas mixture not satisfying the quality criterion takes place fully, with the exception of the decoupling of an amount of gas otherwise exceeding the intake capacity of the method, and in particular takes place absolutely fully.

11. The method according to any of the preceding claims, wherein the quality criterion has one or a number of sub-criteria which are chosen from the group:
i) minimum methane content, particularly preferably at least 90%, more preferably at least 92%, in particular at least 95%,
ii) maximum carbon dioxide content, particularly preferably maximum 12%, more preferably maximum 8%, in particular maximum 4%,
iii) maximum hydrogen content, particularly preferably maximum 4%, more preferably maximum 2%, in particular maximum 1%.

12. The method according to any of the preceding claims, wherein at least some of the hydrogen of the reactant gas is generated by electrolysis.

13. The method according to any of the preceding claims, wherein the electric energy for generating hydrogen originates from excess currents occurring in the power grid and/or regenerative energy sources, in particular temporally variably available energy sources such as wind and/or solar energy.

14. The method according to any of the preceding claims, wherein the transition between the operating modes takes place on average at least in the range of several times a week, in particular once or also a number of times a day.

15. An arrangement for providing a methane-rich product gas comprising:
a catalytically active methanation reactor (R),
a feed device (1, 2) upstream of the methanation reactor for feeding a reactant gas having a hydrogen and carbon dioxide into the reactor (R), and
a gas outlet (18) downstream of the reactor (R) for providing the gas mixture generated in the reactor (R) and satisfying a specified quality criterion as regards the gas composition as the product gas,
**characterised by** a utilisation device (3, 4; 3, 6; 13) coupled between the reactor outlet and the gas outlet for decoupling and at least partially recycling a gas mixture passing out of the reactor (R) and not satisfying the quality criterion, wherein the utilisation device has a refeed device (3, 4; 3, 6) for at least partially refeeding the gas mixture not satisfying the quality criterion to a feed point located in front of the reactor inlet and wherein the refeed device has intermediate storage (4), as well as by a control device (30) controlling the arrangement to execute a method according to any of claims 1 to 14.

16. The arrangement according to claim 15, wherein the refeed device has a line arrangement (7) to the feed bypassing the reactor of at least one reactant gas component into the intermediate storage.

17. The arrangement according to claim 16, wherein the line arrangement has a separate supply line (7.1) from the H₂ and/or a separate supply line (7.2) from the CO₂ source of the feed device.

18. The arrangement according to any of claims 15 to 17, wherein the refeed device has a measuring device (5) measuring the gas composition in the intermediate storage.

19. The arrangement according to any of claims 15 to 18, wherein the utilisation device has a gas separation device (13) for separating a gas portion worsening the gas mixture as regards the quality criterion.

20. The arrangement according to any of claims 15 to 19, wherein a connection is provided for a supply line (101) to a gas utilisation unit (100), in particular also the supply line (101) and/or the gas utilisation unit (100) itself.

## Revendications

1. Procédé d'approvisionnement en produit gazeux riche en méthane, dans lequel un gaz de départ comportant de l'hydrogène et du dioxyde de carbone est méthanisé par catalyse, dans un premier mode de fonctionnement, pour obtenir un mélange gazeux qui remplit un critère de qualité prédéfini concernant sa composition en gaz et est approvisionné en tant que ledit produit gazeux, et dans lequel la méthanisation catalytique, dans un deuxième mode de fonctionnement, est diminuée, et notamment arrêtée, par diminution, et notamment par l'arrêt, de l'apport de gaz de départ, en sachant qu'on opère une transition entre les deux modes de fonctionnement en fonction de la disponibilité de l'hydrogène,
**caractérisé en ce qu'**
un mélange gazeux produit lors de la transition et ne remplissant pas le critère de qualité est recyclé au moins partiellement dans le cadre dudit procédé d'approvisionnement par son renvoi au moins partiel à une étape de procédé située en amont de la méthanisation, le renvoi étant retardé dans le temps par le biais d'un stockage intermédiaire du gaz.

2. Procédé selon la revendication 1, dans lequel le mélange gazeux renvoyé est conditionné, par mélange avec un ou plusieurs composants du gaz de départ, pour présenter une composition souhaitée au début de la méthanisation.

3. Procédé selon la revendication 2, dans lequel le conditionnement s'effectue pendant le stockage intermédiaire du gaz.

4. Procédé selon la revendication 3, dans lequel la portion non méthane du mélange gazeux renvoyé est essentiellement rendue conforme à la composition du gaz de départ par le biais dudit conditionnement.

5. Procédé selon la revendication 4, dans lequel le retard temporel est maintenu jusqu'à ce que la conformité de la composition du mélange gazeux soit avérée.

6. Procédé selon l'une des revendications 1 à 5, dans lequel, dans le premier mode de fonctionnement, l'apport en gaz de départ est ajusté, du point de vue stoechiométrique et/ou du débit volumique, en fonction de la composition et/ou du débit volumique du mélange gazeux renvoyé.

7. Procédé selon l'une des revendications précédentes, dans lequel au moins un composant du gaz de départ est approvisionné par une installation de biogaz.

8. Procédé selon la revendication 7, dans lequel le composant du gaz de départ approvisionné par l'installation de biogaz est constitué à partir d'un gaz très riche en carbone séparé lors d'un traitement de biogaz, notamment au moyen d'un lavage aux amines.

9. Procédé selon l'une des revendications précédentes, dans lequel le recyclage se produit, partiellement ou totalement, par séparation d'une portion remplissant ledit critère de qualité et son approvisionnement en tant que ledit produit gazeux et/ou par alimentation du mélange gazeux ne remplissant pas les exigences de qualité dans une unité d'utilisation de gaz, notamment une chaudière à gaz ou une centrale de cogénération.

10. Procédé selon l'une des revendications précédentes, dans lequel le recyclage du mélange gazeux ne remplissant pas le critère de qualité, à l'exception d'une extraction de la quantité de gaz dépassant autrement la capacité de réception du procédé, s'effectue en totalité, notamment en complète totalité.

11. Procédé selon l'une des revendications précédentes, dans lequel le critère de qualité présente un ou plusieurs sous-critères choisis dans le groupe constitué par:
i) la teneur minimale en méthane, celle-ci étant notamment de préférence d'au moins 90%, plus préférablement d'au moins 92%, notamment d'au moins 95%,
ii) la teneur maximale en dioxyde de carbone, notamment de préférence d'au maximum 12%, plus préférablement d'au maximum 8%, notamment d'au maximum 4%,
iii) la teneur maximale en hydrogène, notamment de préférence d'au maximum 4%, plus préférablement d'au maximum 2%, notamment d'au maximum 1%.

12. Procédé selon l'une des revendications précédentes, dans lequel au moins une partie de l'hydrogène du gaz de départ est produite par électrolyse.

13. Procédé selon l'une des revendications précédentes, dans lequel l'énergie électrique nécessaire à la production d'hydrogène provient de courants excédentaires du réseau électrique et/ou de sources d'énergie régéneratives, notamment de sources d'énergie disponibles de manière variable dans le temps telles que l'énergie éolienne et/ou solaire.

14. Procédé selon l'une des revendications précédentes, dans lequel la transition entre les modes de fonctionnement se produit en moyenne au moins environ plusieurs fois par semaine, notamment une fois voire plusieurs fois par jour.

15. Système d'approvisionnement d'un produit gazeux riche en méthane comportant:
un réacteur de méthanisation (R) à action catalytique,
une unité d'amenée (1, 2) située en amont du réacteur de méthanisation et destinée à l'apport d'un gaz de départ comprenant de l'hydrogène et du dioxyde de carbone dans le réacteur (R), et
une sortie de gaz (18) située en aval du réacteur (R) et destinée à l'approvisionnement, en tant que ledit produit gazeux, du mélange gazeux produit dans le réacteur (R) et remplissant un critère de qualité prédéfini concernant la composition en gaz,
**caractérisé par** une unité d'utilisation (3, 4; 3, 6; 13) couplée entre la sortie du réacteur et la sortie de gaz pour l'extraction et le recyclage au moins partiel d'un mélange gazeux sortant du réacteur (R) et ne remplissant pas le critère de qualité, l'unité d'utilisation présentant un dispositif de renvoi (3, 4; 3, 6) permettant le renvoi au moins partiel du mélange gazeux ne remplissant pas le critère de qualité vers un point d'amenée situé en amont de l'entrée du réacteur et le dispositif de renvoi présentant un accumulateur intermédiaire (4), et en plus par un dispositif de commande (30) conçu pour commander le système à exécuter un procédé selon l'une des revendications 1 à 14.

16. Système selon la revendication 15, dans lequel le dispositif de renvoi présente un agencement de conduite (7) permettant l'apport, en contournant le réacteur, d'au moins un composant du gaz de départ dans l'accumulateur intermédiaire.

17. Système selon la revendication 16, dans lequel l'agencement de conduite présente une conduite séparée (7.1) partant de la source de H₂ et/ou une conduite séparée (7.2) partant de la source de CO₂ de l'unité d'amenée.

18. Système selon l'une des revendications 15 à 17, dans lequel le dispositif de renvoi présente une unité de mesure (5) qui mesure la composition en gaz présente dans l'accumulateur intermédiaire.

19. Système selon l'une des revendications 15 à 18, dans lequel l'unité d'utilisation présente une unité de séparation de gaz (13) permettant de séparer une portion gazeuse altérant le mélange gazeux du point de vue du critère de qualité.

20. Système selon l'une des revendications 15 à 19, dans lequel il est prévu un raccord pour une conduite (101) menant à une unité d'utilisation de gaz (100), notamment la conduite (101) et/ou l'unité d'utilisation de gaz (100) elle-même.
